(19) Europäisches Patentamt
European Patent Office
Office européen des brevets

(11) Veröffentlichungsnummer : **0 019 109**
**B1**

(12) # EUROPÄISCHE PATENTSCHRIFT

(45) Veröffentlichungstag der Patentschrift : **15.12.82**

(51) Int. Cl.³ : **C 07 C125/073**, C 07 C125/077

(21) Anmeldenummer : **80102170.0**

(22) Anmeldetag : **23.04.80**

(54) **Verfahren zur Herstellung von aromatischen Di- und Polyurethanen.**

(30) Priorität : **30.04.79 DE 2917569**

(43) Veröffentlichungstag der Anmeldung : **26.11.80 (Patentblatt 80/24)**

(45) Bekanntmachung des Hinweises auf die Patenterteilung : **15.12.82 Patentblatt 82/50**

(84) Benannte Vertragsstaaten : **AT BE CH DE FR GB IT LI NL SE**

(56) Entgegenhaltungen :
**US A 2 409 712**
**US A 2 806 051**

(73) Patentinhaber : **BASF Aktiengesellschaft**
**Carl-Bosch-Strasse 38**
**D-6700 Ludwigshafen (DE)**

(72) Erfinder : **Merger, Franz, Dr**
**Max-Slevogt-Strasse 25**
**D-6710 Frankenthal (DE)**
Erfinder : **Towae, Friedrich, Dr.**
**Parkstrasse 22**
**D-6700 Ludwigshafen (DE)**

**EP 0 019 109 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von aromatischen Di- und Polyurethanen

Die Erfindung betrifft ein Verfahren zur Herstellung von aromatischen Di- und/oder Polyurethanen aus primären aromatischen Di- und/oder Polyaminen, Harnstoff und Alkoholen bei erhöhten Temperaturen, gegebenenfalls unter vermindertem oder erhöhtem Druck und gegebenenfalls in Gegenwart von Katalysatoren.

N-Arylurethane werden technisch üblicherweise durch Umsetzung von Alkoholen mit aromatischen Isocyanaten oder von aromatischen Aminen mit Chlorkohlensäureestern hergestellt, wobei sowohl die Isocyanate als auch die Chlorkohlensäureester durch Phosgenierung der entsprechenden Amine und Abspaltung von Chlorwasserstoff oder durch Phosgenierung der Alkohole gewonnen werden. (Methoden der organischen Chemie, Houben-Weyl, Band 8, Seiten 137, 120 und 101, Georg Thieme Verlag, Stuttgart, 1952). Diese Verfahren sind technisch sehr aufwendig ; ferner bringt die Verwendung von Phosgen wegen damit verbundener Sicherheits- und Umweltschutzmaßnahmen erhebliche Nachteile mit sich.

N-Arylurethane finden als Zwischen- und Endprodukte Verwendung. Gemäß DE-OS 26 35 490 oder US-PS 3 919 278 sind sie beispielsweise zur Herstellung von Isocyanaten geeignet. Andere Herstellungsverfahren für N-substituierte Urethane gewinnen daher zunehmend an Bedeutung.

So beschreibt die DE-OS 21 60 111 ein Verfahren zur Herstellung von N-substituierten Urethanen durch Umsetzung eines organischen Carbonats mit einem primären oder sekundären aromatischen Amin in Gegenwart einer Lewissäure. Nachteilig an diesem Verfahren ist, daß die Reaktionsgeschwindigkeiten recht gering und die Reaktionszeiten dementsprechend groß sind ; außerdem werden als Nebenprodukte zusätzlich stets N-Alkyl-arylamine erhalten.

Nach Angaben der US-PS 2 834 799 werden Carbamidsäure- und Kohlensäureester durch Umsetzung von Harnstoffen mit Alkoholen in Gegenwart von Bortrifluorid hergestellt. Nachteilig ist hierbei, daß das Bortrifluorid als Katalysator in äquimolaren Mengen benötigt wird, so daß pro erzeugtes Molekül Carbamidsäureester mindestens ein und pro Molekül Kohlensäureester mindestens zwei Moleküle Bortrifluorid verbraucht werden. Dadurch gestaltet sich das Verfahren nicht nur teuer, sondern es stellt auch eine erhebliche Umweltbelastung dar, da das Bortrifluorid in Form des $H_3N \cdot BF_3$-Adduktes anfällt.

Methyl-N-phenylurethan kann ferner aus Kohlenmonoxid, Schwefel, Anilin und Methanol hergestellt werden (R.A. Franz et al, J. Org. Chem. *28*, 585 (1963)). Nachteilig sind hierbei insbesondere die geringen Ausbeuten, die auch bei langen Reaktionszeiten nicht über 25 % liegen.

Nach Angaben der US-PS 2 409 712 können N-Alkyl- und N-Arylmonourethane durch Umsetzung von Monoaminen mit Harnstoff, N,N'-Dialkyl- oder N,N'-Diarylharnstoff und Alkoholen bei Temperaturen von 150 °C bis 350 °C, gegebenenfalls unter erhöhtem Druck, hergestellt werden. Beispielhaft beschrieben wird jedoch lediglich die Herstellung von N-Alkylmonourethanen nach der genannten Methode, während N-Phenylurethan durch Alkoholyse von Diphenylharnstoff hergestellt wird.

Zur Herstellung von N-substituierten Monourethanen wird der Harnstoff gemäß US-PS 2 677 698 zunächst mit Monoaminen in den entsprechenden N,N'-disubstituierten Harnstoff übergeführt, dieser wird gereinigt und anschließend mit Alkohol zur Reaktion gebracht.

Nachteilig an den genannten Verfahren sind neben einer aufwendigen Technik insbesondere die geringen Ausbeuten, die auch durch die Herstellung und Reinigung von N,N'-disubstituierten Harnstoffen nicht verbessert werden können.

Diese Nachteile können auch mit Hilfe des Verfahrens nach US-PS 2 806 051 nicht beseitigt werden. Nach Angaben dieser Patentschrift wird beispielsweise Anilin mit Harnstoff und Alkohol im Molverhältnis 1,0 : 1,2 : 2,0 bei Temperaturen unter 200 °C, vorzugsweise von 120° bis 160 °C umgesetzt. Auch in den vorzugsweise genannten Temperaturbereichen werden in technisch zweckmäßigen Reaktionszeiten nach dieser Verfahrensweise N-Phenylmonourethane nur in geringen Ausbeuten erhalten. Es ist daher nicht überraschend, daß in der nachgängigen US-PS 3 076 007 zur Herstellung von N-Alkyl- und N-Cycloalkylurethanen die obengenannten Methoden unter Verwendung von gegebenenfalls substituierten Harnstoffen nicht beschrieben werden ; erwähnt wird hingegen die Umsetzung von Phosgen mit Alkoholen zu Chloralkylformiaten und die anschließende Weiterreaktion mit Aminen zu Urethanen, während als besonders vorteilhaft zur Herstellung der Urethane die Reaktion von Aminen mit Äthylencarbonat empfohlen wird. Nach einer neueren Verfahrensvariante (DE-OS 27 16 540) werden zur Herstellung von aromatischen Urethanen Dialkylcarbonate mit N-Acylaminen zur Reaktion gebracht.

Verfährt man analog den Angaben der Beispiele der US-PS 2 806 051, setzt jedoch anstelle von Monoaminen Diamine um, so erhält man dem beschriebenen Stand der Technik entsprechend mit hoher Ausbeute in Form eines Niederschlags ein Produkt, dessen Struktur weitgehend identisch ist mit den bekannten Polyharnstoffen aus Diaminen und Polyisocyanaten. Dies verdeutlicht ebenfalls, daß das Verfahren gemäß US-PS 2 806 051 auf die Umsetzung von Monoaminen beschränkt ist. Es ist daher nicht überraschend, daß in den genannten Patentschriften die Herstellung von Di- und Polyurethanen, ausgehend von schwach basischen aromatischen Di- und/oder Polyaminen, nach diesem Verfahrensprinzip nicht erwähnt wird.

Die Aufgabe der vorliegenden Erfindung bestand darin, aromatische Di- und/oder Polyurethane aus leicht zugänglichen Ausgangskomponenten, möglichst in einer Reaktionsstufe unter wirtschaftlich

vertretbaren Bedigungen in guten Ausbeuten herzustellen. Die Verwendung von stark toxischen Ausgangsstoffen, beispielsweise von Phosgen, Kohlenmonoxid, oder Katalysatoren, die toxisch sind oder im Laufe der Umsetzung toxische Verbindungen bilden, beispielsweise Schwefelwasserstoff, sollte weitgehend vermieden werden.

Diese Aufgabe wurde gelöst durch ein Verfahren zur Herstellung von aromatischen Di- und/oder Polyurethanen, das dadurch gekennzeichnet ist, daß man ein primäres aromatisches Di- und/oder Polyamin mit Harnstoff und einem Alkohol im Verhältnis von $NH_2$-Gruppen der primären aromatischen Amine zu Harnstoff zu Hydroxylgruppen der Alkohole von 1 : 08-10 : 5-100 gegebenenfalls in Gegenwart von Katalysatoren bei Temperaturen von 160 °C bis 300 °C umsetzt und gegebenenfalls den entstehenden Ammoniak abtrennt.

Die Reaktion kann durch folgende Gleichung veranschaulicht werden :

$$Ar - (NH_2)_n + nH_2NCONH_2 + nHOR \longrightarrow Ar - (NHCOOR)_n + 2nNH_3.$$

Die Bildung von aromatischen Di- und/oder Polyurethanen, insbesondere in einem Verfahrensschritt und in guten Ausbeuten, ist besonders überraschend, da nach bekannter Lehrmeinung aus Harnstoff und aromatischen Monoaminen die entsprechenden N,N'-Diarylharnstoffe, z.B. aus Harnstoff und Anilin N,N'-Diphenylharnstoff, erhalten werden, die meist aus Alkohol umkristallisiert werden. Aus Harnstoff und Alkohol können auch unsubstituierte Urethane erhalten werden, die jedoch in Gegenwart von aromatischen Aminen sehr leicht zu N,N'-disubstituierten Arylharnstoffen weiterreagieren (Methoden der organischen Chemie, Houben-Weyl, Band 8, Seiten 151, 140 und 161, Georg Thieme Verlag, Stuttgart, 1952). Entsprechend werden aus Diaminen und Harnstoff nicht nur N,N'-disubstituierte Harnstoffe, sondern bei Temperaturen über 100 °C hochmolekulare, verspinnbare Kondensationsprodukte erhalten (DE-PS 896 412). Hochmolekulare Polyharnstoffe, beispielsweise mit Molekulargewichten von 8 000 bis 10 000 und größer werden auch erhalten, wenn man Diurethane mit Diaminen bei Temperaturen von ungefähr 150 °C bis 300 °C kondensiert (US-PS 2 181 663 und US-PS 2 568 885). Da Mono- und Polyurethane außerdem thermisch in Isocyanate, Alkohole und gegebenenfalls Olefine, Kohlendioxid, Harnstoff und Carbodiimide gespalten werden, wobei die erhaltenen Spaltprodukte wieder zahlreiche Folgeprodukte z.B. Biurete, Allophanate, Isocyanurate, Polycarbodiimide u.a. bilden können (J.A.C.S. *80,* 5495, (1958) und J.A.C.S. *78,* 1946, (1956)), konnte nicht erwartet werden, daß unter sehr ähnlichen Reaktionsbedingungen nach dem erfindungsgemäßen Verfahren Di- und/oder Polyurethane in sehr guten Ausbeuten erhalten werden.

Dies war besonders überraschend, da Versuche zur Herstellung von Diurethanen aus den obengenannten Produkten nach den erfindungsgemäßen Reaktionsbedingungen nicht zum Ziele führten.

Für die erfindungsgemäße Umsetzung mit Harnstoff und Alkoholen eignen sich gegebenenfalls substituierte primäre aromatische di- und Polyamine oder deren Gemische, wobei die primären aromatischen Diamine bevorzugt verwendet werden. Im einzelnen seien beispielhaft genannt : aromatische Diamine, wie 1,3- und 1,4-Diaminobenzol ; durch eine Nitro-, Methyl-, Äthyl-, n-Propyl-, Isopropyl-, n-Butyl-, Isobutyl-, sek.-Butyl, tert.-Butyl-, Methoxy-, Äthoxy-, n-Propoxy-, Isopropoxy-, n-Butoxy-, Isobutoxy-, sek.-Butoxy-, tert.-Butoxygruppe oder ein Halogenatom, vorzugsweise ein Fluor- und/oder Chloratom, in 2- und/oder 4-Stellung substituiertes 1,3-Diaminobenzol oder in 2-Stellung substituiertes 1,4-Diaminobenzol, 1,5- und 1,8-Diaminonaphthalin, 4,4'-Diaminodiphenyl, 2,2'-, 2,4'- und 4,4'-Diaminodiphenylmethan und die entsprechenden Isomerengemische und Polyphenyl-polymethylen-polyamine sowie Mischungen aus Diamino-diphenylmethanen und Polyphenyl-polymethylen-polyaminen, die nach bekannten Verfahren durch Kondensation von Anilin und Formaldehyd in Gegenwart von vorzugsweise Mineralsäuren als Katalysatoren erhalten werden.

Besonders bewährt haben sich und daher vorzugsweise verwendet werden 2,4- und 2,6-Diaminotoluol sowie die entsprechenden Isomerengemische, 2,2'-, 2,4'- und 4,4'-Diamino-diphenylmethan und die entsprechenden Isomerengemische, 1,5-Diamino-naphthalin und als Polyamine Mischungen aus Diamino-diphenylmethanen und Polyphenyl-polymethylenpolyaminen.

Als Alkohole für das erfindungsgemäße Verfahren können beliebige, gegebenenfalls substituierte primäre oder sekundäre aliphatische Alkohole und aromatisch-aliphatische Alkohole sowie Mischungen davon verwendet werden. In Betracht kommen beispielsweise primäre aliphatische Monoalkohole mit 1 bis 20 Kohlenstoffatomen, vorzugsweise 1 bis 10 Kohlenstoffatomen, wie Methanol, Äthanol, Propanol, n-Butanol, Isobutanol, 2- und 3-Methylbutanol, Neopentylalkohol, Pentanol, 2-Methyl-pentanol, n-Hexanol, 2-Äthylhexanol, n-Heptanol, n-Octanol, n-Nonanol, n-Decanol, n-Dodecanol, 2-Phenylpropanol und Benzylalkohol, sekundäre aliphatische und cycloaliphatische Monoalkohole mit 3 bis 15 Kohlenstoffatomen, vorzugsweise 3 bis 6 Kohlenstoffatomen, wie Isopropanol, sec.-Butanol, sec.-Isoamylalkohol, Cyclopentanol, Cyclohexanol, 2,3- oder 4-Methyl-cyclohexanol und 4-tert.-Butyl-cyclohexanol. Vorzugsweise verwendet werden die Monoalkohole, Methanol, Äthanol, Propanol, Butanol, Isobutanol, 2- und 3-Methyl-butanol, 2-Äthyl-butanol, Pentanol, 2-Methyl-pentanol, Hexanol, 2-Äthylhexanol, Heptanol, Octanol und Cyclohexanol.

Zur Herstellung der aromatischen Di- und/oder Polyurethane nach dem erfindungsgemäßen Verfahren werden die obengenannten primären aromatischen Di- und/oder Polyamine und Alkohole mit dem Harnstoff in solchen Mengen zur Reaktion gebracht, daß das Verhältnis von $NH_2$-Gruppe der

aromatischen Di- und Polyamine zu Harnstoff zu Hydroxylgruppe der Alkohole 1 : 0,8-10 : 5-100, vorzugsweise 1 : 0,9-7 : 5-50 und insbesondere 1 : 1-5 : 10-30 beträgt.

Die Umsetzung wird vorzugsweise in Gegenwart von überschüssigem Alkohol als Lösungs- und Reaktionsmittel und in Abwesenheit oder gegebenenfalls in Gegenwart von Katalysatoren bei erhöhten Temperaturen und gegebenenfalls unter verminderten oder erhöhtem Druck durchgeführt, wobei es sich als vorteilhaft erwiesen hat, den entstehenden Ammoniak sofort aus der Reaktionsmischung, beispielsweise durch Destillation, abzutrennen.

Wie bereits dargelegt wurde, wird die Umsetzung vorzugsweise in einem Alkoholüberschuß durchgeführt, so daß der Alkohol als Reaktionskomponente und gleichzeitig als Lösungsmittel fungiert. Anstelle von Alkohol können jedoch auch Mischungen aus Alkoholen und anderen unter den Reaktionsbedingungen inerten organischen Lösungsmitteln verwendet werden.

Die Herstellung der aromatischen Di- und/oder Polyurethane nach dem erfindungsgemäßen Verfahren wird zweckmäßigerweise in Abwesenheit von Katalysatoren durchgeführt, sofern die Umsetzung der ausgewählten Reaktionspartner in wirtschaftlich tragbaren Reaktionszeiten mit guten Ausbeuten erfolgt. Auf diese Weise werden kostspielige Reinigungsoperationen zur Abtrennung der Katalysatoren aus den erhaltenen Endprodukten vermieden.

Wird zur Erhöhung der Reaktionsgeschwindigkeit, vorzugsweise bei niedrigen Temperaturen, die Umsetzung in Gegenwart von Katalysatoren durchgeführt, so werden diese zweckmäßigerweise in Mengen von 0,1 bis 20 Gew.%, vorzugsweise 0,5 bis 10 Gew.%, und insbesondere 1 bis 5 Gew.%, bezogen auf das Gewicht des aromatischen primären Amins, verwendet. Als Katalysatoren eignen sich anorganische oder organische Verbindungen, die ein order mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th. Edition, publiziert von Chemical Rubber Publishing Co. 2310 Superior Ave. N.E. Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielhaft genannt seien die Kationen folgender Metalle : Lithium, Natrium, Kalium, Magnesium, Calcium, Aluminium, Gallium, Zinn, Blei, Wismut, Antimon, Kupfer, Silber, Gold, Zink, Quecksilber, Cer, Titan, Vanadium, Chrom, Molybdän, Mangan, Eisen, Kobalt und Nickel. Vorzugsweise Verwendung finden die Kationen von Lithium, Calcium, Aluminium, Zinn, Wismut, Antimon, Kupfer, Zink, Titan, Vanadium, Chrom, Molybdän, Mangan, Eisen und Kobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt : Lithiummethanolat, Lithiumäthanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Wismut-(III)-chlorid, Kupfer-(II)-acetat, Kupfer-(II)-sulfat, Kupfer-(II)-nitrat, Bis-(triphenylphosphinoxido)-kupfer-(II)-chlorid), Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphthenat, Vanadium-(III)-chlorid, Vanadiumacetonylacetat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Kobaltacetat, Kobaltchlorid, Kobaltsulfat, Kobaltnaphthenat, Nickelchlorid, Nickelacetat und Nickelnaphthenat sowie deren Gemische.

Die Umsetzung wird bei erhöhten Temperaturen, beispielsweise bei Temperaturen von 160° bis 300 °C, vorzugsweise von 180° bis 250 °C und insbesondere von 185° bis 230 °C und Drücken von 0,1 bis 120 bar, vorzugsweise 0,5 bis 60 bar, insbesonders 1 bis 40 bar, durchgeführt. Für diesen Temperaturbereich ergeben sich Reaktionszeiten von 0,1 bis 50 Stunden, vorzugsweise von 0,5 bis 20 Stunden. Bei gegebener Temperatur wird die Reaktion dann vorzugsweise unter einem Druck durchgeführt, bei dem der entstehende Ammoniak selektiv aus dem Reaktionsgemisch abdestilliert werden kann. Die entsprechenden Werte können Tabellen mit physikalischen Kenndaten des Ammoniak und der Alkohole entnommen werden.

Nach dem erfindungsgemäßen Verfahren werden die Di- und/oder Polyurethane zweckmäßigerweise wie folgt hergestellt : Die Ausgangskomponenten werden in den entsprechenden Molverhältnissen gemischt und gegebenenfalls in Gegenwart von Katalysatoren in einem Reaktionsgefäß oder gegebenenfalls einem Druckgefäß, ausgestattet mit einer Vorrichtung zur Abtrennung des Ammoniaks, erhitzt. Der entstehende Ammoniak kann nach beendeter Umsetzung abgetrennt werden ; vorzugsweise wird er jedoch bereits im Laufe der Umsetzung abdestilliert. Hierbei kann es zweckmäßig sein, insbesondere bei der Umsetzung von niedermolekularen Alkoholen unter Druck, den Ammoniak mit Hilfe eines unter den Reaktionsbedingungen inerten Schleppmittels, beispielsweise eines Gases, wie Stickstoff, abzutrennen.

Nach einer anderen Ausführungsform, die im allgemeinen zu einer erheblichen Reduzierung der Reaktionszeit führt und in diesen Fällen vorteilhafterweise angewandt wird, werden die primären aromatischen Di- und/oder Polyamine, der Harnstoff und der Alkohol zunächst in einem Verhältnis $NH_2$-Gruppen der Amine zu Harnstoff zu Alkohol von 1 : 1,5-3 : 2-10, vorzugsweise 1 : 1,5-2 : 4-8 0,5 bis 3, vorzugsweise 0,5 bis 2 Stunden zur Reaktion gebracht, danach wird der Reaktionsmischung zusätzlicher

Alkohol in einer solchen Menge einverleibt, daß pro eingesetzter $NH_2$-Gruppe der Amine 10 bis 30, vorzugsweise 15 bis 30 Mol Alkohol vorliegen, und die Reaktion in einem Zeitraum von insgesamt 4 bis 20 Stunden, vorzugsweise 4 bis 10 Stunden zu Ende geführt. Aus der erhaltenen Reaktionsmischung wird anschließend, gegebenenfalls nach Abtrennung des Katalysators, und/oder Abfiltrieren von Feststoffen das Di- und/oder Polyurethan isoliert, beispielsweise durch Abdestillieren des Alkohols und/oder des Lösungsmittels sowie von gegebenenfalls als Nebenprodukt gebildeten O-Alkylcarbamaten, durch partielles Abdestillieren des Alkohols und Auskristallisieren, durch Auskristallisieren, durch Ausfällen mit oder auch durch Umkristallisieren aus anderen Lösungsmitteln.

Die erhaltenen aromatischen di- und/oder Polyurethane sind wertvolle End- und Zwischenprodukte. Sie werden beispielsweise als Schädlingsbekämpfungsmittel verwendet. Als Zwischenprodukte werden sie als Komponenten für Polykondensations- und Polymersysteme eingesetzt, insbesondere jedoch unter Abspaltung von Alkohol in die entsprechenden Isocyanate übergeführt, wobei Di- und Polyisocyanate zur Herstellung von Polyurethanen Anwendung finden.

Die in den Beispielen genannten Teile beziehen sich auf das Gewicht. Die Elementarzusammensetzungen und Strukturen sind durch Elementaranalyse, Massenspektroskopie sowie IR- und NMR-Spektren bestätigt.

Beispiel 1

10 Teile 4,4'-Diamino-diphenylmethan werden mit 15,2 Teilen Harnstoff in 195 Teilen n-Octanol 5 Stunden auf Rückflußtemperatur (ungefähr 195 °C) erhitzt und hierbei gleichzeitig der entstehende Ammoniak abdestilliert. Man läßt abkühlen und filtriert das auskristallisierte 4,4'-Di-(octoxycarbonylamino)-diphenylmethan ($C_{31}H_{46}O_4N_2$, Molgewicht 510) ab. Ausbeute : 24,2 Teile (94,0 % der Theorie). Schmelzpunkt : 118-119 °C.

Beispiel 2

12,2 Teile 2,4-Diamino-toluol werden mit 60 Teilen Harnstoff in 390 Teilen n-Octanol 7 Stunden lang auf Rückflußtemperatur (ungefähr 195 °C) erhitzt und hierbei gleichzeitig der entstehende Ammoniak abdestilliert. Danach wird die Reaktionslösung bis zu einer Sumpftemperatur von 180 °C fraktioniert destilliert, wobei nacheinander überschüssiges Octanol und unsubstituierter Carbamidsäureester zurückgewonnen werden. Man erhält einen Rückstand, in dem nach der Methode des « Externen Standards » durch Verwendung der Hochdruck-Flüssigkeitschromatographie 38,6 Teile 2,4-Di-(octoxycarbonylamino)-toluol (88,9 % der Theorie), $C_{25}H_{42}O_4N_2$ (Molekulargewicht 434) nachgewiesen werden. Schmelzpunkt (aus Cyclohexan) : 68-70 °C.

Beispiel 3

5 Teile 4,4'-Diamino-diphenylmethan werden mit 6,1 Teilen Harnstoff und 46 Teilen Butanol zum Sieden erhitzt, wobei über ein Druckventil im Reaktionsgefäß ein Druck von 7 bis 8 bar eingestellt wird, so daß die Siedetemperatur des Butanols bei ca. 200 °C liegt. Der während der Reaktion gebildete Ammoniak wird unter Verwendung von 25 l pro Stunde Stickstoff je Liter Reaktionsgemisch als Strippgas abdestilliert. Man läßt abkühlen, entspannt und engt das Lösungsmittel ein. Hierbei kristallisieren 8,4 Teile 4,4'-Di-(butoxycarbonylamino)-diphenylmethan (83,6 % der Theorie), $C_{23}H_{30}O_4N_2$ (Molekulargewicht 398) aus. Schmelzpunkt/91-95 °C.

In der Reaktionslösung findet man neben Ausgangsprodukt noch 4-Amino-4'-(butoxycarbonylamino)-diphenylmethan.

Beispiel 4

12,2 Teile 2,4-Diamino-toluol werden mit 14 Teilen Harnstoff und 200 Teilen Cyclohexanol 10 Stunden zum Sieden erhitzt, wobei über ein Druckventil im Reaktionsgefäß ein Druck von 2 bis 3 bar eingestellt wird, so daß die Siedetemperatur des Cyclohexanols bei ca. 200 °C liegt. Der während der Reaktion gebildete Ammoniak wird unter Verwendung von 25 l/Std. Stickstoff je Liter Reaktionsgemisch als Strippgas kontinuierlich abdestilliert. Nach dem Erkalten wird das Gemisch durch Hochdruck-Flüssigchromatographie nach der Methode des « Externen Standards » analysiert. Hierbei zeigte sich, daß 18 Teile 2,4-Di-(octoxycarbonylamino)-toluol (48,1 % der Theorie) und 12,4 Teile eines Gemisches aus 2-Amino-4-(octoxycarbonylamino)-toluol und 4-Amino-2-(octoxycarbonylamino)-toluol (50,0 % der Theorie) gebildet werden, so daß die Gesamtausbeute an Urethanen 98,1 % beträgt.

Beispiel 5

8 Teile 1,5-Diamino-naphthalin werden mit 18 Teilen Harnstoff in 150 Teilen n-Octanol 10 Stunden auf Rückflußtemperatur (ungefähr 195 °C) erhitzt und gleichzeitig der entstehende Ammoniak abgetrennt. Man läßt abkühlen ; hierbei kristallisiert ein Gemisch aus nicht umgesetztem 1,5-Diamino-naphthalin und

1,5-Di-(octoxycarbonylamino)-naphthalin, $C_{28}H_{42}O_4N_2$ (Molekulargewicht 470) aus. Nach Umkristallisieren aus Essigester erhält man 15 Teile 1,5-Di-(octoxycarbonylamino)-naphthalin vom Schmelzpunkt 69-71 °C (64,3 % der Theorie). Die Mutterlaugen enthalten noch 1,5-Diamino-naphthalin und 1-Amino-5-(octoxycarbonylamino)-naphthalin.

Beispiel 6

8 Teile 1,5-Diamino-naphthalin werden mit 12 Teilen Harnstoff in 57 Teilen Äthanol 18 Stunden zum Sieden erhitzt, wobei über ein Druckventil im Reaktionsgefäß ein Druck von 22 bis 24 bar eingestellt wird, so daß die Siedetemperatur des Äthanols bei ca. 190 °C liegt. Der während der Reaktion gebildete Ammoniak wird unter Verwendung von 30 l/Std. Stickstoff je Liter Reaktionsgemisch als Strippgas kontinuierlich abdestilliert. Man läßt erkalten, entspannt das Reaktionsgemisch und destilliert den Alkohol ab. Hierbei kristallisieren 11,7 Teile 1,5-Di-(äthoxycarbonylamino)-naphthalin, $C_{16}H_{18}O_4N_2$ (Molgewicht 302 ; 76,5 % der Theorie) mit einem Schmelzpunkt von 222-224 °C aus.

Beispiel 7

5 Teile eines handelsüblichen Gemisches aus 2,2'-, 2,4'- und 4,4'-Diamino-diphenylmethanen und Polyphenyl-polymethylen-polyaminen werden mit 4 Teilen Harnstoff in 93 Teilen n-Octanol zum Sieden erhitzt. Man läßt abkühlen, destilliert überschüssiges Octanol und gebildeten Carbamidsäureoctylester bis zu einer Sumpftemperatur von 180 °C ab und verrührt den Rückstand mit 50 Teilen Cyclohexan. Man erhält einen pulverförmigen Niederschlag, der von der Lösung abgetrennt wird. Die Analyse des Niederschlags mit Hilfe der Hochdruck-Flüssigchromatographie ergibt, daß 7,6-Teile eines Gemisches aus 2,2'-, 2,4'- und 4,4'-Di-(octoxycarbonylamino-diphenylmethanen sowie Polyphenyl-polymethylenpolyoctylurethane gebildet wurden.

Vergleichsbeispiel (analog US-PS 2 806 051)

10 Teile 4,4'-Diamino-diphenylmethan werden mit 7,3 Teilen Harnstoff und 15 Teilen n-Butanol zum Sieden erhitzt. Nach etwa 45 Minuten beginnt sich die Reaktionsmischung zu trüben ; während der weiteren Umsetzung fallen beträchtliche Mengen eines unlöslichen Feststoffes aus, welcher im Verlauf von weiteren 90 Stunden nicht mehr gelöst wird. Aufgrund der IR-Analyse muß dem erhaltenen Feststoff Polyharnstoffstruktur zugeschrieben werden.

Beispiel 8

Man verfährt analog den Angaben von Beispiel 5, fügt der Reaktionsmischung jedoch zusätzlich 0,1 Teile Natriummethylat als Katalysator hinzu. Man erhält 18,7 Teile 1,5-Di-(octoxycarbonylamino)-naphthalin (78,6 % der Theorie), $C_{28}H_{42}O_4N_2$, (Molekulargewicht 470) mit einem Schmelzpunkt von 71-72 °C.

Beispiel 9

12,2 Teile 2,4-Diamino-toluol werden mit 32 Teilen Harnstoff in 104 Teilen n-Octanol zum Sieden erhitzt. Nach 60 Minuten fügt man zu der Reaktionsmischung zusätzlich 416 Teile n-Octanol und kocht weitere 4 Stunden. Während der gesamten Reaktion wird kontinuierlich Ammoniak abdestilliert. Nach beendeter Umsetzung werden überschüssiges n-Octanol und als Nebenprodukt gebildeter Carbamidsäure-octylester bis zu einer Sumpftemperatur von 190 °C fraktioniert abdestilliert. Im erhaltenen Destillationsrückstand werden 40,2 Teile 2,4-Di-(octoxycarbonyl-amino)-toluol (92,6 % der Theorie) durch Hochdruck-Flüssigchromatographie nach der Methode des « Externen Standards » nachgewiesen.

Beispiel 10

6,1 Teile 2,4-Diamino-toluol werden mit 15 Teilen Harnstoff und 18,4 Teilen Äthanol zum Sieden erhitzt, wobei über ein Druckventil im Reaktionsgefäß ein Druck von 24 bis 25 bar eingestellt wird, so daß die Siedetemperatur des Äthanols bei ungefähr 195 °C liegt. Nach einer Reaktionszeit von 45 Minuten werden dem Reaktionsgemisch zusätzlich 74 Teile Äthanol einverleibt. Der während der Umsetzung gebildete Ammoniak wird mit Hilfe von 30 l/Std. Stickstoff je Liter Reaktionsgemisch kontinuierlich abdestilliert. Man läßt das Reaktionsgemisch abkühlen und entspannt. Beim Abkühlen in einer Eis-Kochsalzmischung kristallisieren 7,9 Teile 2,4-Di-(äthoxycarbonylamino)-toluol (59,4 % der Theorie), $C_{13}H_{18}O_4N_2$ (Molekulargewicht 266), mit einem Schmelzpunkt von 109-110 °C aus. Mit Hilfe der Hochdruck-Flüssigchromatographie nach der Methode des « Externen Standards » werden in der Mutterlauge 3,2 Teile 2,4-Di-(äthoxycarbonylamino)-toluol (24,1 % der Theorie) und 1,4-Teile eines Gemisches von 2-Amino-4-(äthoxycarbonylamino)-toluol und 4-Amino-2-(äthoxycarbonylamino)-toluol (14,4 % der Theorie) nachgewiesen, so daß die Gesamtausbeute an Urethanen 97,9 % beträgt.

Beispiel 11

6,1 Teile 2,4-Diaminotoluol werden mit 12 Teilen Harnstoff und 18,4 Teilen Äthanol zum Sieden erhitzt, wobei über ein Druckventil im Reaktionsgefäß ein Druck von 17 bis 18 bar eingestellt wird, so daß die Siedetemperatur der Reaktionsmischung bei ca. 180° liegt. Der während der Reaktion gebildete Ammoniak wird mit Hilfe von 5 Litern Stickstoff pro Stunde und je Liter Reaktionsgemisch kontinuierlich abdestilliert. Mit Hilfe der Hochdruckflüssigchromatographie nach der Methode des « Externen Standard » werden in der Reaktionslösung nach 5 Stunden 6,2 Teile 2,4-Di-(äthoxycarbonylamino)-toluol nachgewiesen, was einem Umsatz von 2,4-Diaminotoluol von 79,3 % und einer Ausbeute an Diurethan von 58,8 % mit einer Raum-Zeit-Ausbeute von 34,0 g/l · h entspricht.

Beispiele 12 bis 16

Man verfährt analog den Angaben von Beispiel 11, fügt der Reaktionsmischung jedoch zusätzlich 0,1 Teile eines Katalysators bei.

Die verwendeten Katalysatoren, die Reaktionszeiten und Ausbeuten sind in der folgenden Tabelle zusammengefaßt :

TABELLE

| Beispiel Nr. | Katalysator | Umsatz % | Zeit h | Raum-Zeit-Ausbeute g/l · h | Ausbeute % |
|---|---|---|---|---|---|
| 12 | Kobalt-(II)-acetat | 83,2 | 5 | 38,8 | 64,0 |
| 13 | Mangan-(II)-acetat | 81,9 | 5 | 39,3 | 65,8 |
| 14 | Vanadiumtrichlorid | 96,1 | 3 | 55,1 | 47,2 |
| 15 | Zink-(II)-acetat | 78,5 | 3 | 65,1 | 68,3 |
| 16 | Eisen-(II)-acetat | 77,8 | 3 | 63,5 | 67,2 |

**Ansprüche**

1. Verfahren zur Herstellung von aromatischen Di- und/oder Polyurethanen, dadurch gekennzeichnet, daß man ein primäres aromatisches Di- und/oder Polyamin mit Harnstoff und einem Alkohol im Verhältnis von $NH_2$-Gruppen der primären aromatischen Amine zu Harnstoff zu Hydroxylgruppen der Alkohole von 1 : 0,8 bis 10 : 5 bis 100 gegebenenfalls in Gegenwart von Katalysatoren bei Temperaturen von 160 bis 300 °C umsetzt und gegebenenfalls den entstehenden Ammoniak abtrennt.

2. Verfahren gemäß Anspruch 1, dadurch gekennzeichnet, daß man gleichzeitig den entstehenden Ammoniak abtrennt.

3. Verfahren gemäß Anspruch 1, dadurch gekennzeichnet, daß man die Umsetzung bei Drücken von 0,1 bis 120 bar durchführt.

4. Verfahren gemäß Anspruch 1, dadurch gekennzeichnet, daß man als aromatische Diamine 2,4- und 2,6-Diamino-toluol und die entsprechenden Isomerengemische, 1,5-Diamino-naphthalin, 2,2'-, 2,4'- und 4,4'-Diamino-diphenylmethan und die entsprechenden Isomerengemische und als Polyamine Mischungen aus Diaminodiphenylmethanen und Polyphenylpolymethylenpolyaminen verwendet.

5. Verfahren gemäß Anspruch 1, dadurch gekennzeichnet, daß man als Alkohole aliphatische und cycloaliphatische Alkohole verwendet.

6. Verfahren nach Anspruch 5, dadurch gekennzeichnet, daß man als Alkohole Methanol, Äthanol, Propanol, Butanol, Isobutanol, 2- und 3-Methylbutanol, 2-Äthylbutanol, Pentanol, 2-Methylpentanol, Hexanol, 2-Äthylhexanol, Heptanol, Octanol und Cyclohexanol verwendet.

7. Verfahren gemäß Anspruch 1, dadurch gekennzeichnet, daß man die Ausgangskomponenten im Verhältnis $NH_2$-Gruppen der Amine zu Harnstoff zu Alkohol von 1 : 1,5 bis 3 : 2 bis 10 0,5 bis 3 Stunden lang kondensiert, der Reaktionsmischung zusätzlichen Alkohol in einer Menge einverleibt, daß das Verhältnis $NH_2$-Gruppen der Amine zu Alkohol 1 : 10 bis 30 beträgt und die Reaktion zu Ende führt.

8. Verfahren gemäß Anspruch 1, dadurch gekennzeichnet, daß man als Katalysatoren Verbindungen verwendet, die ein oder mehrere Kationen von Metallen aus den Gruppen IA, IB, IIA, IIB, IIIA, IIIB, IVA, IVB, VA, VB, VIB, VIIB und VIIIB des Periodensystems gebunden enthalten.

**Claims**

1. A process for the preparation of an aromatic di- and/or polyurethane, characterized in that a

primary aromatic di- and/or polyamine is reacted with urea and an alcohol at temperatures of from 160 °C to 300 °C in the presence or absence of a catalyst, the ratio of $NH_2$ groups of the primary aromatic amines to urea to hydroxyl groups of the alcohol being 1 : 0.8-10 : 5-100, and the ammonia which is formed is optionally separated off.

2. A process in claimed in claim 1, characterized in that the ammonia is separated off as it is formed.

3. A process as claimed in claim 1, characterized in that the reaction is carried out at pressures of from 0.1 bar to 120 bars.

4. A process as claimed in claim 1, characterized in that 2,4- and 2,6-diaminotoluene, the corresponding isomer mixtures thereof, 1,5-diaminonaphthalene, 2,2'-2,4'- and 4,4'-diaminodiphenylmethane and the corresponding isomer mixtures thereof are used as aromatic diamines, and mixtures of diaminophenylmethanes and polymethylene polyphenylamines are used as polyamines.

5. A process as claimed in claim 1, characterized in that an aliphatic or cycloaliphatic alcohol is used as the alcohol.

6. A process as claimed in claim 5, characterized in that methanol, ethanol, propanol, butanol, isobutanol, 2- and 3-methylbutanol, 2-ethylbutanol, pentanol, 2-methylpentanol, hexanol, 2-ethylhexanol, heptanol, octanol or cyclohexanol is used as the alcohol.

7. A process as claimed in claim 1, characterized in that the starting components are condensed in a ratio of $NH_2$ groups of the amines to urea to alcohol of 1 : 1.5-3 : 2-10 for 0.5 to 3 hours, and additional alcohol is incorporated into the reaction mixture in such an amount that the ratio of $NH_2$ groups of the amines to alcohol is 1 : 10-30 and the reaction is completed.

8. A process as claimed in claim 1, characterized in that a compound containing, in combined form, one or more cations of metals selected from groups IA, IB, IIA, IIB, IIIA, IVA, IVB, VA, VIB, VIIB and VIIIB of the periodic system is used as the catalyst.

**Revendications**

1. Procédé de préparation de di- et/ou polyuréthanes aromatiques, caractérisé par le fait qu'on fait réagir, à des températures de 160 à 300 °C, éventuellement en présence de catalyseurs, une di- et/ou polyamine aromatique primaire avec de l'urée et un alcool, dans les rapports, entre groupes $NH_2$ de l'amine aromatique primaire, urée et groupes hydroxyle de l'alcool, de 1/0,8 à 10/5 à 100 et on sépare éventuellement l'ammoniac produit.

2. Procédé selon la revendication 1, caractérisé par le fait que l'on sépare simultanément l'ammoniac produit.

3. Procédé selon la revendication 1, caractérisé par le fait que l'on effectue la réaction à des pressions de 0,1 à 120 bars.

4. Procédé selon la revendication 1, caractérisé par le fait que l'on utilise, comme diamines aromatiques, le 2,4- et 2,6 diaminotoluène et les mélanges isomères correspondants, le 1,5-diaminonaphtalène, les 2,2'-, 2,4'- et 4,4'-diaminophénylméthane et les mélanges isomères correspondants et, comme polyamines, des mélanges de diaminodiphénylméthanes et polyphényl-polyméthylène-polyamines.

5. Procédé selon la revendication 1, caractérisé par le fait que l'on utilise, comme alcools, des alcools aliphatiques et cycloaliphatiques.

6. Procédé selon la revendication 5, caractérisé par le fait que l'on utilise, comme alcools, méthanol, éthanol, propanol, butanol, isobutanol, 2- et 3-méthylbutanol, 2-éthylbutanol, pentanol, 2-méthylpentanol, hexanol, 2-éthylhexanol, heptanol, octanol et cyclohexanol.

7. Procédé selon la revendication 1, caractérisé par le fait que l'on condense, pendant 0,5 à 3 heures, les composants de départ dans les rapports, entre groupes $NH_2$ de l'amine, urée et alcool, de 1/1,5 à 3/2 à 10, on introduit dans le mélange de réaction de l'alcool additionnel en quantité telle que le rapport entre groupes $NH_2$ de l'amine et l'alcool soit de 1/10 à 30 et on mène la réaction à sa fin.

8. Procédé selon la revendication 1, caractérisé par le fait que l'on utilise, comme catalyseurs, des composés qui contiennent, liés, un ou plusieurs cations de métaux des groupes IA, IB, IIA, IIB, IIIA, IIIB, IVA, IVB, VA, VB, VIB, VIIB et VIIIB du système périodique.